# EUROPEAN PATENT SPECIFICATION

(11) **EP 1 904 113 B1**
(45) Date of publication and mention of the grant of the patent: **06.01.2016**
(21) Application number: 06785518.9
(22) Date of filing: 23.06.2006
(51) Int. Cl.: A61K 48/00, A61K 38/18

(54) **NEUROPROTECTIVE EFFECTIVE COMPOUND**
NEUROPROTEKTIVE WIRKSAME VERBINDUNG
COMPOSÉ NEUROPROTECTEUR EFFICACE

(30) Priority: 23.06.2005 US 595342 P
(43) Date of publication of application: 02.04.2008
(73) Proprietor: TissueGene, Inc., Gaithersburg, MD 20877 (US)
(72) Inventor: LEE, Kwan, Hee, Rockville, MD 20850 (US); LEE, Duk, Keun, Rockville, MD 20850 (US); YI, Youngsuk, Rockville, MD 20850 (US); NOH, Moon, Jong, Rockville, MD 20850 (US); KIM, Hyoung, Chun, Chunchon 200-701 (KR)
(74) Representative: Fiener, Josef
(86) International application number: PCT/US2006/024656
(87) International publication number: WO 2007/002512

(56) References cited:
- WO-A-01/17547
- WO-A-02/07749
- WO-A-95/05846
- WO-A-96/25929
- WO-A-03/082302
- WO-A-2006/002202
- WO-A1-03/056925
- WO-A2-02/07774
- US-B1- 6 593 105
- PARK KUN-WOO ET AL: "Protection of nigral neurons by GDNF-engineered marrow cell transplantation" NEUROSCIENCE RESEARCH, vol. 40, no. 4, August 2001 (2001-08), pages 315-323, XP002510385 ISSN: 0168-0102
- WINN S R ET AL: "POLYMER-ENCAPSULATED CELLS GENETICALLY MODIFIED TO SECRETE HUMAN NERVE GROWTH FACTOR PROMOTE THE SURVIVAL OF AXOTOMIZED SEPTAL CHOLINERGIC NEURONS" PROCEEDINGS OF THE NATIONAL ACADEMY OF SCIENCES OF USA, NATIONAL ACADEMY OF SCIENCE, WASHINGTON, DC.; US, vol. 91, no. 6, 6 March 1994 (1994-03-06), pages 2324-2328, XP001076846 ISSN: 0027-8424
- DUMONT R.J. ET AL.: 'Ex vivo bone morphogenic protein-9 gene therapy using human mesenchymal stem cells induces spinal fusion in rodents' NEUROSURGERY vol. 51, no. 5, 2002, pages 1239 - 1245, XP008069932
- LAWRENCE J.M. ET AL.: 'Transplantation of Schwann cell line clones secreting GDNF or BDNF into the retinas of dystrophic royal college of surgeons rats' INVEST. OPHTHALMAL. VIS. SCI. vol. 45, no. 1, January 2004, pages 267 - 274, XP003015184
- BRECKNELL J.E. ET AL.: 'Bridge grafts of fibroblast growth factor-4 secreting Schwannoma cells promote functional axonal regeneration in the nigrostriatal pathway of the adult rat' NEUROSCIENCE vol. 74, no. 3, 1996, pages 775 - 784, XP003015185
- TUSZYNSKI M H: "Growth-factor gene therapy for neurodegenerative disorders", LANCET NEUROLOGY, LANCET PUBLISHING GROUP, LONDON, GB, vol. 1, no. 1, 1 May 2002 (2002-05-01), pages 51-57, XP004814216, ISSN: 1474-4422, DOI: 10.1016/S1474-4422(02)00006-6
- HARRINGTON K ET AL: "Cells as vehicles for cancer gene therapy: the missing link between targeted vectors and systemic delivery?", HUMAN GENE THERAPY, MARY ANN LIEBERT, NEW YORK ,NY, US, vol. 13, no. 11, 20 July 2002 (2002-07-20) , pages 1263-1280, XP002242755, ISSN: 1043-0342, DOI: 10.1089/104303402760128504

## Description

### BACKGROUND OF THE INVENTION

### 1. Field of the Invention:

The present invention relates to prevention of nerve degeneration.

### 2. General Background and State of the Art:

There is a need in the art for a molecular therapeutic neuroprotective compound for preventing the degradation of the neural system and neuronal cells.

WO 2006/002202 A discloses cells expressing BMP-2 and BMP-9 for use in regenerating nerves, being different from slowing-down the degeneration of nerves.
WO 02/07749 A discloses a composition for stimulating nerve cell growth. Nerve growth polymeric conduits are described, but not for slowing-down the degeneration of nerve damage.

NEUROSCIENCE RESEARCH, vol. 40, no. 4, pages 315 - 323, August 2001, (PARK et al.) discloses injecting bone marrow cells transduced with GDNF gene, in which the cells are seen to travel to the brain for action.
WO 01/17547 A discloses BMP-9 compositions that act as a differentiating factor for the cholinergic phenotype of CNS neurons.

WO 95/05846 A describes a method for preventing degeneration of a nerve by administering a composition comprising a BMP protein.
WO 03/082302 A refers to irradiating cells expressing BMP prior to their transplantation.

The Lancet Neurology, Vol. 1, pp. 51-57, May 2002, (Tuscynski) discloses NGF gene therapy, but not for use in treating neurodegenerative disease or epilepsy.
WO 02/07774 A discloses *in vivo* neurotrophin gene therapy, but not fot treating diseases as epilepsy or neurodegenerative disease.

The same applies for WO03/056925 A, referring to *in vivo* BDNF, NT-4/5 and NT-3 gene therapy. Human Gene Therapy 13:1263-1280, July 2002, (Harrington et al.), merely discloses using cells as carriers.

### SUMMARY OF THE INVENTION

The invention is directed to a population of cultured cells transfected *in vitro* with a generated recombinant viral or plasmid vector comprising a DNA sequence encoding a bone morphogenetic protein BMP operatively linked to a promoter.

In particular, as exemplified in the present application, the cells are neuroprotective against the cytotoxic effects of amphetamine or kainic acid.

The cell may be a connective tissue cell, such as a fibroblast cell. The cell may also be a nerve cell, such as glial cell. The cell may be irradiated. The vector may be a viral vector. The viral vector may be a retroviral vector, adeno-associated viral vector, adenoviral vector, or herpes viral vector.

The invention is directed to preventing degeneration of nerve, comprising administering to an area near an injured nerve a population of cells comprising a DNA sequence encoding a BMP protein. The BMP protein may be BMP-2, BMP-3, BMP-4 or BMP-9.

In addition, the population of cells may be stored prior to transplantation such as in 10% DMSO under liquid nitrogen.

### BRIEF DESCRIPTION OF THE DRAWINGS

The present invention will become more fully understood from the detailed description given herein below, and the accompanying drawings which are given by way of illustration only, and thus are not limitative of the present invention, and wherein;

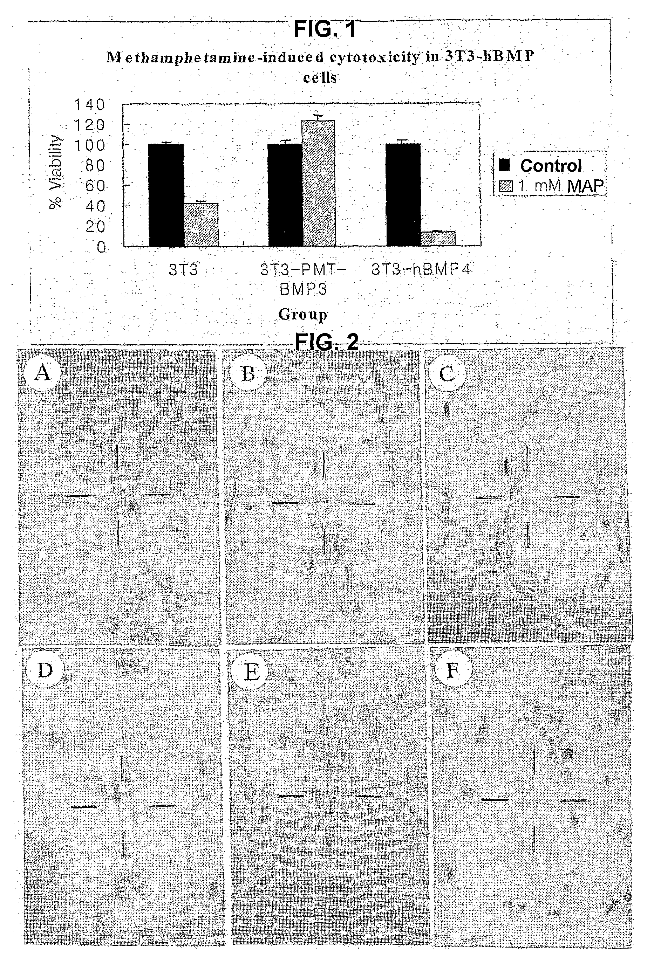

FIGURE 1 shows methamphetamine (MAP)-induced cytotoxicity in 3T3-hBMP cells. Closed box shows 3T3 cells, 3T3-PMT-BMP3, or 3T3-hBMP4 cells without any treatment with MAP. The hatched boxes show treatment with 1mM MAP.
FIGURES 2A-2F show microscopic photographs of cells corresponding to those observed in FIGURE 1. FIGS. 2A, 2B and 2C correspond to the control 3T3 cells, 3T3-PMT-BMP3, or 3T3-hBMP4 cells that have not been treated with MAP. FIGS. 2D, 2E and 2F correspond to the control 3T3 cells, 3T3-PMT-BMP3, or 3T3-hBMP4 cells, respectively, that have been treated with 1mM MAP as indicated above in FIG. 1.

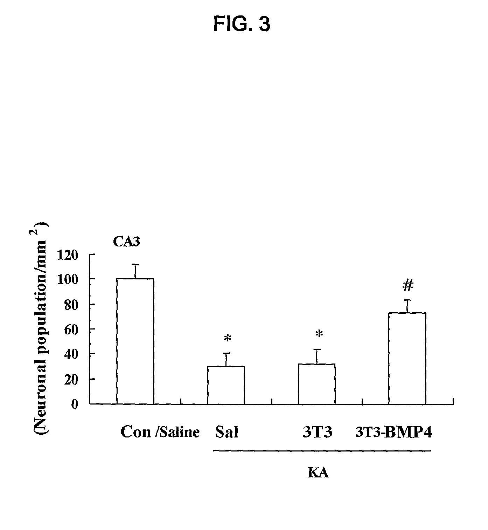

FIGURE 3 shows effects of NIH3T3-BMP4 on the neuronal loss induced by kainite (I.C.V. 0.1 µg/head) in mice. Each value is the mean +/- S.E.M. of 4 animals. *p<0.001 vs. con, #p<0.01 vs. Sal + KA or 3T3+KA (ANOVA with DMR test).

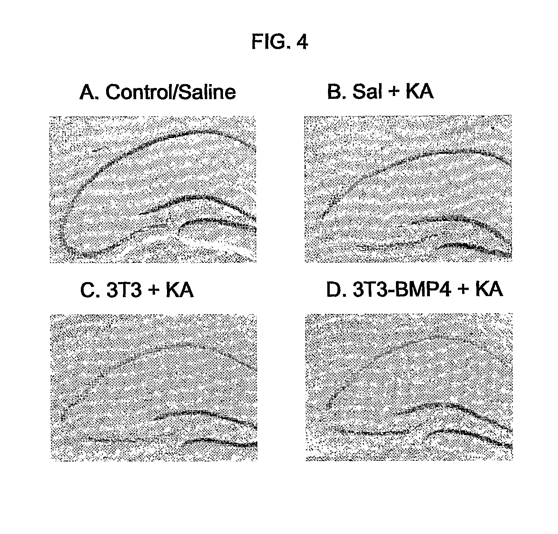

FIGURES 4A-4D show microscopic photographs of neuronal loss as a result of treatment of mice with kainite. FIG. 4A shows hippocampal CA3 section of animals treated with Control Saline solution; FIG. 4B shows CA3 section of animals treated with Saline + Kainate solution; FIG. 4C shows CA3 section of animals treated with NIH3T3 alone before being injected with Kainate; and FIG. 4D shows CA3 section of animals treated with NIH3T3 cells expressing recombinant BMP4 before being injected with Kainate.

### DETAILED DESCRIPTION OF THE PREFERRED EMBODIMENTS

In the present application, "a" and "an" are used to refer to both single and a plurality of objects.

As used herein, the term "connective tissue cell" or "cell of a connective tissue" include cells that are found in the connective tissue, such as fibroblasts, cartilage cells (chondrocytes), and bone cells (osteoblasts/ osteocytes), which secrete collagenous extracellular matrix, as well as fat cells (adipocytes) and smooth muscle cells. Preferably, the connective tissue cells are fibroblasts, cartilage cells, and bone cells. More preferably, the connective tissue cells are fibroblast cells. Connective tissue cells also include mesenchymal cells, which are also known as immature fibroblasts. It will be recognized that the invention can be practiced with a mixed culture of connective tissue cells, as well as cells of a single type.

As used herein, injection of cells "near" an injured nerve or neural system is meant that area which is close enough between the injection site and the injury area to effect an efficacious outcome of preventing degeneration of the injured nerve cells at the injured site. Therefore, the injection of cells near an injured nerve includes at the site of injury or anywhere close enough for the injected cells to express the effective polypeptide and the polypeptides are allowed to directly or indirectly effect the nerve degeneration preventing outcome. For peripheral nerve, especially in spinal cord injury, the injection can be made "upstream" of the injury site since cells tend to leak out at the site of injury.

As used herein, a "promoter" can be any sequence of DNA that is active, and controls transcription in an eucaryotic cell. The promoter may be active in either or both eucaryotic and procaryotic cells. Preferably, the promoter is active in mammalian cells. The promoter may be constitutively expressed or inducible. Preferably, the promoter is inducible. Preferably, the promoter is inducible by an external stimulus. More preferably, the promoter is inducible by hormones or metals. Still more preferably, the promoter is inducible by heavy metals. Most preferably, the promoter is a metallothionein gene promoter. Likewise, "enhancer elements", which also control transcription, can be inserted into the DNA vector construct, and used with the construct of the present invention to enhance the expression of the gene of interest.

As used herein, "selectable marker" includes a gene product that is expressed by a cell that stably maintains the introduced DNA, and causes the cell to express an altered phenotype such as morphological transformation, or an enzymatic activity. Isolation of cells that express a transfected gene is achieved by introduction into the same cells a second gene that encodes a selectable marker, such as one having an enzymatic activity that confers resistance to an antibiotic or other drug. Examples of selectable markers include, but are not limited to, thymidine kinase, dihydrofolate reductase, aminoglycoside phosphotransferase, which confers resistance to aminoglycoside antibiotics such as kanamycin, neomycin and geneticin, hygromycin B phosphotransferase, xanthine-guanine phosphoribosyl transferase, CAD (a single protein that possesses the first three enzymatic activities of *de novo* uridine biosynthesis - carbamyl phosphate synthetase, aspartate transcarbamylase and dihydroorotase), adenosine deaminase, and asparagine synthetase (Sambrook et al. Molecular Cloning, Chapter 16. 1989).

As used herein, the "transforming growth factor-β (TGF-β) superfamily" encompasses a group of structurally related proteins, which affect a wide range of differentiation processes during embryonic development. The family includes, Müllerian inhibiting substance (MIS), which is required for normal male sex development (Behringer, et al., Nature, 345:167, 1990), Drosophila decapentaplegic (DPP) gene product, which is required for dorsal-ventral axis formation and morphogenesis of the imaginal disks (Padgett, et al., Nature, 325:81-84, 1987), the Xenopus Vg-1 gene product, which localizes to the vegetal pole of eggs (Weeks, et al., Cell, 51:861-867, 1987), the activins (Mason, et al., Biochem, Biophys. Res. Commun., 135:957-964, 1986), which can induce the formation of mesoderm and anterior structures in Xenopus embryos (Thomsen, et al., Cell, 63:485, 1990), and the bone morphogenetic proteins (BMP's, such as BMP-2, 3, 4, 5, 6 and 7, osteogenin, OP-1) which can induce *de novo* cartilage and bone formation (Sampath, et al., J. Biol. Chem., 265:13198, 1990). The TGF-β gene products can influence a variety of differentiation processes, including adipogenesis, myogenesis, chondrogenesis, hematopoiesis, and epithelial cell differentiation (for a review, see Massague, Cell 49:437, 1987).

The proteins of the TGF-β family are initially synthesized as a large precursor protein, which subsequently undergoes proteolytic cleavage at a cluster of basic residues approximately 110-140 amino acids from the C-terminus. The C-terminal regions of the proteins are all structurally related and the different family members can be classified into distinct subgroups based on the extent of their homology. Although the homologies within particular subgroups range from 70% to 90% amino acid sequence identity, the homologies between subgroups are significantly lower, generally ranging from only 20% to 50%. In each case, the active species appears to be a disulfide-linked dimer of C-terminal fragments. For most of the family members that have been studied, the homodimeric species has been found to be biologically active, but for other family members, like the inhibins (Ung, et al., Nature, 321:779, 1986) and the TGF-β's (Cheifetz, et al., Cell, 48:409, 1987), heterodimers have also been detected, and these appear to have different biological properties than the respective homodimers.

Members of the superfamily of TGF-β genes include TGF-β3, TGF-β2, TGF-β4 (chicken), TGF-β1, TGF-β5 (*Xenopus*), BMP-2, BMP-3, BMP-4, BMP-5, BMP-6, OP-1/BMP-7, BMP-8, BMP-9, *Drosophila* 60A, *Drosophila* DPP, Vgr1, GDF-1, *Xenopus* Vgf, Inhibin-βA, Inhibin-βB, Inhibin-α, and MIS. Many of these genes are discussed in Massague, Ann. Rev. Biochem. 67:753-791, 1998.

Preferably, the member of the superfamily of TGF-β genes is TGF-β. More preferably, the member is TGF-β1, TGF-β2, TGF-β3, BMP-2, BMP-3, BMP-4, BMP-5, BMP-6, BMP-7, BMP-8, or BMP-9.

It is understood that in describing a protein by its designated name as above, the protein is not limited to the exact sequence of the wild-type. Variations on the sequence of the protein is acceptable such that another polypeptide sequence, which exhibits substantially the same activity as the protein in terms of function is encompassed thereby.

### Nerve Tissue

Nervous tissue derives from the embryonic ectoderm under the influence of the notochord. The ectoderm is induced to form a thickened neural plate that then differentiates and the ends eventually fuse to form the neural tube from which all of the central nervous system derives. The central nervous system consists of the brain, cranial nerves and spinal cord. The peripheral nervous system derives from cells next to the neural groove called the neural crest.

Nerve tissue is distributed throughout the body in a complex integrated communications network. Nerve cells (neurons) communicate with other neurons in circuits ranging form very simple to very complex higher-order circuits. Neurons do the actual message transmission and integration while other nervous tissue cells called glial cells assist neurons by support, protection, defense and nutrition of the neurons. There are about 10 times more glial cells than neurons in the brain. Glial cells create the microenvironment needed for neuronal function and sometimes they assist in neural processing and activity. Neurons are excitable cells. This means that when properly stimulated, an action potential can be initiated that may be propagated over the cell membrane to transmit information to distant cells. Neurons are independent functional units responsible for the reception, transmission and processing of stimuli.

In general, neurons consist of three parts; the cell body, where the nucleus and cellular organelles are located; dendrites, which are processes extending from the cell body that receive stimuli from the environment or other neurons; and the axon, which is a long single process extending from the cell body for the transmission of nerve impulses to other cells. The axon usually branches at its distal end and each branch terminating on another cell has a bulbous end. The interaction of the end bulb with the adjacent cell forms a structure called a synapse. Synapses are specialized to receive a signal and convert it into an electrical potential.

Most neurons found in the human body are multipolar, meaning they have more than two cell processes with only one being an axon and the remaining processes being dendrites. Bipolar neurons of the retina or olfactory mucosa have one dendritic process and an axon coming off the cell body. Pseudounipolar neurons found in the spinal cord ganglia enable sensory impulses picked up by the dendrites to travel directly to the axon without passing through the cell body. Neurons may also be classified according to function. Sensory neurons are involved in the reception and transmission of sensory stimuli. Motor neurons send impulses to control muscles and glands. Other neurons, interneurons, act as go-betweens between neurons as part of functional networks.

Synapses are specialized functional cell junctions to propagate cellular signals. Most synapses are chemical synapses where vesicles in the presynaptic terminal contain a chemical messenger that is released to the synaptic cleft when the presynaptic membrane is stimulated. The chemical messenger diffuses across the synaptic cleft to bind to receptors in the postsynaptic membrane. This induces a change in the polarization state of the postsynaptic membrane effecting cellular action. A special type of synapse is the neuromuscular junction. More than 35 neurotransmitters are known and most are small molecules (nitric oxide, acetylcholine), catecholamines (norepinephrine, serotonin), or neuroactive peptides (endorphin, vasopressin). Once used, the neurotransmitters are removed quickly by enzymatic breakdown, diffusion or endocytosis by the presynaptic cell.

Some neurons are wrapped in an insulating material called myelin. This lipid rich material is formed by glial cells: Schwann cells in the peripheral nervous system and by oligodendrocytes in the central nervous system. The insulation enables faster nerve conduction by reducing the membrane surface area that must be depolarized. In myelinated neurons the nerve impulse jumps from one unmyelinated segment to another over the length of the axon. It is the myelin sheath and lack of neuron cell bodies within the tissue that makes some nervous tissue appear white as in the large peripheral nerves and white matter of the brain. Other glial cells, called astrocytes, are involved in structural integrity, neuronal nutrition and maintaining the microenvironment of nervous tissue. Astrocytes, are in direct communication with one another via gap junctions and can affect the survival of neurons in their care by the regulation of the the local environment. Ependymal cells line spinal cord and the ventricles of the brain and secrete the cerebrospinal fluid. Other small glial cells, called microglia, are phagocytic cells that are involved with inflammation and repair in the adult central nervous system.

Nervous tissue is an excitable tissue that is capable of receiving and transmitting electrical impulses. The central cell type is called a neuron. Neurons usually have a cell body, dendrites that receive inputs, and an axon that transmits electrical potentials.

Neurons may be classified as sensory, motor, secretory or association neurons. They are often classified by conduction speed, diameter and the presence or absence of specialized lipoprotein insulation called myelin. Type A fibers are myelinated and can conduct impulses at 12 -120 m/sec. Type B are also myelinated fibers but they only transmit impulses at 3-5 m/sec. Type C fibers are unmyelinated, small in diameter and very slow (2.5 m/sec). An example of a Type A fiber is a motor neuron innervating the gastrocnemius. An autonomic preganglionic efferent neuron is an example of a Type B fiber and a sensory neuron carrying information about diffuse pain is an example of a slow Type C fiber.

Sensory neurons are adapted to detect certain types of information from the environment. These include mechanoreceptors sensing things like pressure or stretch, thermoreceptors, photoreceptors in the retina, and chemoreceptors such as the taste bud or those for olfaction. Association neurons, or interneurons are usually found in the spinal cord and brain where they connect sensory afferent neurons to efferent motor or secretory neurons.

Neurons communicate with one another via a structure called the synapse. An axon ends in one or more terminal buttons that contain numerous small vesicles. These small vesicles are filled with chemical substances called neurotransmitters. Acetylcholine is most often the neurotransmitter at the synapse although other chemicals like norepinephrine, serotonin and GABA may be used dependent on the neuron. When an impulse travels down the axon and reaches the terminal buttons the vesicles fuse with the neuronal membrane and the neurotransmitter is released. The chemical then diffuses across the narrow synaptic cleft to specific receptors for the chemical on the postsynaptic membrane of the receiving neuron.

The interaction of the neurotransmitter with the receptor causes a change in the membrane potential that may induce a new impulse postsynaptic neuron. The enzyme acetylcholinesterase is present in synapse to break down acetycholine and terminate the stimulus. Other neurotransmitters are either broken down or taken back up into the presynaptic neuron to terminate the stimulus.

In the central nervous system many neurons may converge on a single neuron. When each of the presynaptic neurons releases neurotransmitter into its synapse with the postsynaptic neuron, local membrane potentials occur that are integrated and summed. These incoming signals may be inhibitory or stimulatory. If the resulting summed membrane potential reaches the minimum threshold for that neuron, then an action potential will be initiated.

Action potentials travel in one direction away from the cell body by saltatory conduction. The fastest neurons are covered in myelin sheaths arranged in discreet segments separated by nodes of naked neuronal membrane called nodes of Ranvier. In saltatory conduction, the electrical potential jumps from node to node, thereby reducing the membrane area involved in conduction of the action potential and speeding up conduction.

Non-neural cells found in the nervous system are called glial cells. Astrocytes are the most numerous and provide support and nourishment of neurons. Microglia are small phagocytic cells specific to neural tissue. Cells that line the ventricular system and central canal of the spinal cord and make cerebrospinal fluid are called ependymal cells. In the central nervous system, an oligodendrocyte forms segments of the myelin sheaths of multiple neurons. In the peripheral nervous system, each segment of the myelin sheath is made by a single Schwann cell.

### Central nervous system

The central nervous system (CNS) consists of the brain and spinal cord. The meninges (dura mater, arachnoid and pia mater) protect and nourish the CNS in addition to the protection afforded by the bony skull and vertebrae. Cerebrospinal fluid is found in the the subarachnoid space, central canal of the spinal column and the ventricles of the brain. The pia mater is the innermost layer and is adherant to the nervous tissue. Between the pia mater and the dura mater lies the arachnoid layer. The tough fibrous dura mater lies just beneath the skull.

The brain can be divided into 3 basic areas of the forebrain, midbrain, and brain stem. The forebrain includes the thalamus, hypothalamus, basal ganglia, and cerebrum. The cerebrum is responsible for conscious thought, interpretation of sensations, all voluntary movements, mental faculties, and the emotions.

Cerebral tissue can be divided into structural and functional areas. The surface of the cerebrum is convoluted into gyri (ridges) and sulci (grooves). The cortical sensory and motor areas can be mapped to the post central gyrus and central sulcus, respectively. The sensory area receives sensory info from the opposite side of the body that is projected after thalamic processing. Those parts of the body with more sensory nerve endings are represented by more cortical sensory area. The motor area controls voluntary muscle movements of the contralateral body parts but the association areas are important for the initiation of movement.

The cerebrum is the largest part of the brain and is divided into two hemispheres, right and left, having several lobes. The frontal lobe contains the motor area, Broca's speech area, association areas, and functions in intelligence and behavior. The parietal lobe contains sensory areas and function in feeling and hearing. Primary visual association areas are located in the occipital lobe and the temporal lobe contains areas for auditory association, smell and memory storage.

The thalamus is located between the cerebral cortex and brainstem. All sensory input except the sense of smell is processed here before being projected to other areas of the brain. The hypothalamus is located beneath the thalamus and is responsible for processing internal stimuli and the maintenance of the internal environment. Moment by moment unconscious control of blood pressure, temperature, heart rate, respiration, water metabolism, osmolality, hunger, and neuroendocrine activities are handled here. Nuclei of the neuroendocrine cells that release oxytocin and ADH from the posterior pituitary are located in the hypothalamus.

The basal ganglia (caudate nucleus, globus palladus, substantia nigra, subthalamic nucleus, red nucleus) are groups of neurons embedded within each hemisphere of the cerebrum. They are involved in the control of complex motor control, information processing and unconscious gross intentional movements.

The brainstem includes the medulla oblongata and pons. The medulla oblongata contains important functional areas and relay centers for the control of respiration, cardiac and vasomotor reflexes. The pons contains the pneumotaxic center which is involved in the regulation of respiration.

The cerebellum lies above the brainstem and uses sensory information processed elsewhere about the position of the body, movement, posture and equilibrium. Movements are not initiated in the cerebellum but it is necessary for coordinated movement.

### Peripheral nervous system

The peripheral nervous system includes nerves, ganglia, spinal and cranial nerves located outside the brain and spinal cord. The twelve cranial nerves arise from nuclei located in the brainstem and travel to specific locations carrying impulses to control various autonomic functions like smell, vision, salivation, heart rate and cutaneous sensation. Cranial nerves are often mixed in that they carry sensory and motor components but they may have only motor or sensory fibers. The following table lists the cranial nerves and their functions.

**Table 1 - Cranial Nerves**

| **Number** | **Name** | **Function** |
|---|---|---|
| I | Olfactory | Sense of smell |
| II | Optic | Vision |
| III | Oculomotor | Motor control of some eye muscles and eyelid |
| IV | Trochlear | Motor control of some eye muscles |
| V | Trigeminal | Chewing muscles and some facial sensation |
| VI | Abducent | Motor control of some eye muscles |
| VII | Facial | Motor control of facial muscles, salivation. Taste and cutaneous sensations. |
| VIII | Acoustic | Equilibration, static sense and hearing |
| IX | Glossopharyngeal | Salivation, sensations of skin, taste and viscera |
| X | Vagus | Motor control of the heart and viscera, sensation from the thorax, pharynx and abdominal viscera |
| XI | Accessory | Motor impulses to the pharynx and shoulder |
| XII | Hypoglossal | Motor control of the tongue, some skeletal muscles, some viscera, sensation from skin and viscera |

The sensory division of the peripheral nervous system takes input from various types of receptors, processes it and sends to the central nervous system. Sensory input can come from internal sources as in proprioception (sense of position of the joints and muscles) or external sources as in the sensation of pressure or heat on the skin. Areas of the skin innervated by specific spinal nerves are called dermatomes. Afferent fibers collect sensory input and travel up the spinal cord, converge in the thalamus, and end finally on the sensory cortex of the cerebrum. Those areas with more sensory receptors, i.e. the fingertips or lips, correspond to a larger area on the sensory cortex of the brain. Fibers carrying proprioceptive information are dispersed to the cerebellum as well. Almost all sensory systems transmit impulses to parts of the thalamus. The cerebral cortex is involved in conscious perception and interpretation of sensory stimuli.

Motor inputs to muscles and glands occur via the autonomic and somatic efferent systems. CNS innervation of the joints, tendons and muscles travel via the somatic efferent system. Some muscular responses are handled via spinal reflexes. An example of this is the withdrawal reflex seen when the finger contacts a hot stove. The movement to remove the finger occurs via a simple spinal reflex long before the sensation of pain reaches the brain. Clearly this is protective mechanism to avoid further injury. Motor inputs to glands and smooth muscle usually occur via the autonomic system.

Most organs receive input from both branches of the autonomic nervous system. One branch will generally be excitatory while the other is inhibitory in that organ or tissue. The sympathetic branch of the autonomic system acts to prepare the body for physiologic stress. Stimulation of the sympathetic branch is like stepping on the gas in that the body prepares to run or fight in response. Effects such as an increased heart rate, dilation of airways and mobilization of glucose from glycogen stores are seen. Sympathetic nerves arise from the 1^{st} thoracic to the 4^{th} lumbar vertebra. They have a short preganglionic neuron that ends in one of the chain ganglia that lie along the spinal column. Acetylcholine is the neurotransmitter at the synapse with the long postganglionic neuron which then travels to the target tissue where norepinephrine is released at the majority of sympathetic nerve endings. A few sympathetic post ganglionic neurons, such as those innervating sweat glands or skeletal muscle vasculature, release acetylcholine.

The parasympathetic branch acts to counterbalance the sympathetic branch via neurons that arise from the cranial and sacral regions of the CNS. For instance, parasympathetic stimulation constricts airways and decreases heart rate. It regulates resting activities such as digestion, micturation and erection. Long preganglionic neurons release acetylcholine at synapses close to the end organ. Short postganglionic neurons also release acetylcholine on the effector tissue.

TGF-β, activins, and BMP are proteins involved in cell differentiation, growth and organ formation during development. BMP is a member of the TGF-β superfamily along with growth/differentiation factors (GDF), osteogenic proteins (OPs), and Mullerian inhibiting substance/anti-Mullerian Hormone (MIS/AMH) (Ebara and Nakayama, Spine, 2002, 16S: S10-S15). Historically, in 1965, Urist (Urist, MR: Bone, formation by autoinduction, Science, 1965, 150(698): 893-899) observed the phenomenon of embryonic ossification and other processes similar to endochondral ossification after inserting demineralized bone matrix into the muscle of both rodents and rabbits. After implantation, undifferentiated mesenchymal cells migrated to the inserted bone tissues by chemotaxis, followed by mitosis and condensation. Chondroblasts, which are derived from mesenchymal cells, then secreted extracellular matrix, which enabled formation of the cartilage template. This extracellular matrix is vascularized through hematopoietic and endothelial cells. Osteoblast and osteoclast began to appear locally and absorbed cartilage was transformed into bone tissues. After 21 days, ossicle with marrow core of bone was formed (Wang et al., Proc Nat Acad Sci USA, 1988, 85: 9484-9488). The component, which was associated with this changing process from the demineralized bone matrix, was described as bone morphogenic protein (BMP).

In 1988, Wang et al. (Wang et al., Proc Nat Acad Sci USA, 1988, 85: 9484-9488) isolated three polypeptides with the molecular weights of 16 kDa, 18 kDa, and 30 kDa each from bovine bones. Wozney et al. (Woozney, Mol Rep Dev, 1992, 32: 160-167) later identified human RNAs and corresponding DNAs using these polypeptides as probes. Follow up studies have revealed the presence of at least 16 endogenous BMP's (Wozney and Rosen, Clin Orthop, 1998, 346: 26-37).

Except for BMP1 (procollagen C-protease), they are all members of transforming growth factor (TGF)-β gene superfamily (Wozney and Rosen, Clin Orthop, 1998, 346: 26-37). Structurally, BMPs are produced as a large precursor form composed of a 15-25 amino acid signal peptide, a prodomain with 50-375 amino acids, and mature terminal carboxyl terminal with 100-125 amino acids. The latter has well-conserved 7 cysteine residues, enabling peptide dimerization after the carboxy terminal region is cleaved from the precursor by proteolytic processing (Croteau et al., 1999; 22: 686-695). Each active, mature BMP protein exists as a disulfide-linked homodimer composed of the same monomers or disulfide-linked heterodimer composed of two different types of monomers (Sampath et al., J Biol Chem, 1990, 265: 13198-13250). Interestingly, the dimerization of the protein has been linked to its activity, and as such the heterodimers, BMP2 and BMP7 have been shown to be stronger morphogens than the homodimer composed of the same monomer (Kawabata et al., Cytokine Growth Factor Rev, 1998, 9: 49-61; Sampath et al., J Biol Chem, 1990, 265: 13198-13250).

In order to capitalize on the biological activity of BMP, it is necessary to understand the regulation of BMP gene expression and the mechanism of BMP dimerization in the cell. Although not much is known about the gene expression of BMP, it is known that it may be regulated by basic helix-loop-helix (bHLH) protein (Ebara et al., Biochem Biophys Res Commun, 1997, 240: 136-141). This bHLH protein is composed of 3 domains, with two outer domains acting as positive transcriptional activators and the center domain acting as a negative regulator. Among these domains, the E-box (the DNA Sequence ranging from 246∼265bp) is recognized by the USF transcription factor and has an important role in regulating the expresison of BMP in the mouse. BMP has also been implicated in the regulation of the cell death pathway.

The biological activities of BMP are tightly regulated at various timepoints beyond the transcriptional level, and even extracellularly. It is thought that outside of the cells, receptors of BMP serving as inhibitory proteins, easily react with BMPs and in response to the increased activity of BMP, may elicit enhanced production of negative feedback signals ultimately leading to their regulation (Ebara and Nakayama, Spine, 2002, 16S: S10-S15). Inside, the cells are regulated both by signal transduction and by repressive Smad proteins, meaning that BMP is able to upregulate expression of repressive Smad proteins (Ebara and Nakayama, Spine, 2002,16S: S10-S15).

At the extracellular level, the cells are controlled by BMP binding proteins such as noggin and chondrin, which inhibit binding of BMP to the cell receptor. Twisted gastrulation (Tsg) enhances the function of chondrin (Ebara and Nakayama, Spine, 2002, 16S: S10-S15). Follisatin binds to OP-1/BMP-7 and BMP-4 proteins and represses BMP. (Matzuk et al., Nature, 1995, 374: 360-363).

### Receptor for BMP

BMP binds to two different types (type I and II) of serine-threonine kinase receptors. Two type I receptors and one type II receptor have been confirmed in mammals (Kawabata et al., Cytokine Growth Factor Rev, 1998, 9: 49-61). In mammals, the type I receptor has isoforms A and B and although they are structurally similar, they show different behaviour in their activation of the Smad proteins (Imamura et al., Nature, 1997, 389: 549-551). For signal transduction, type I and II receptors need to form a complex. Type I receptor is activated by type II receptor and the signal is transduced in cells by the type I receptor. The signal in the cells is transduced by Smad proteins. Smad1, Smad5 and Smad8 belong to the same structure and transduce the signal from BMP. Smad2 and Smad3 transduce signals from TGF-β and activin. These Smads form heteromeric complex and translocate into the nucleus to activate various genes. Smad6, Tob, Ski and Smurf1 are involved in negative regulation of these genes. Among these, Smad6 represses the transcription of BMP and also has a role in negative feedback of BMP signaling pathway (Bai et al., J Biol Chem, 2000, 275: 8267-8270). Tob is a member of antiproliferative protein family and is involved in negative regulation of BMP/Smad signal (Yoshida et al., Cell, 2000, 103: 1085-1097). Ski oncoprotein represses the expression of BMP-signaling and BMP-responsive genes, and represses the activation of BMP by directly reacting with Smad complex, which is a characteristic of BMP (Wang et al., Proc Natl Acad Sci USA, 2000, 97: 14394-14399). Smurf1 belongs to Hect family of ubiquitin ligase and inhibits signal transduction of BMP by selectively binding with receptor-regulated Smad (Zuh et al., Nature, 1999,400: 687-693).

Further research on the function of the BMP family of proteins is ongoing and it seems that they are involved in the scheme of basic body formation including nerve system (Farkas et al., J Neurosci, 1999, 92: 227-235), eyes (Mohans et al., Invest Ophthalmol Vis Sci, 1998, 39: 2626-2636), lung, kidney, prostate, reproductive organ and hair follicles during the embryonic stage. For example, the formation of fingers and spaces between fingers has been reported to be due to apoptosis of cells between fingers caused by BMP (Zou and Niswander, Science, 1996, 272: 738).
BMP is involved in formation, differentiation and healing of skeletal system during the embryonic stage. In the skeletal system after birth, BMP is present in the collagen of brain stroma, periosteum cell and mesenchymal cell of matrix filled with blood forming elements. BMP also has been isolated from osteosarcoma and chondrosarcoma (Lianjia and Yan, Clin Orthop, 1990, 257: 249-256). After fracture, BMP is diffused in the absorbed bone matrix, activates osteoprogenitor cells, and in turn produces more BMP. Distribution of BMP depends on the time of the treatment and location of the fracture and may be further complicated due to reciprocal reactions. BMP research has also been carried out in a variety of other tissues to study their protective or regenerative effect, and have been demonstrated to have a protective effect on the function of heart muscle in ischemia and repurfusion of heart muscle (Lefer et al., J Mol Cell Cardiol, 1992, 24: 585-593), on the extended nervous system in experiments inducing cerebral ishemia after injecting BMP into abdominal cavity and regenerative effects on damaged kidneys (Ripamonti and Duneas, Plast Reconstr Surg, 1998, 101: 227-239).

### BMP Protein Therapy

The present specification encompasses administering BMP protein to the site of nerve degeneration to reconstitute the nerve or to prevent its further degeneration. Preferred are BMP-2, BMP-3, BMP-4, BMP-9.

### Therapeutic Composition

This specification relates to treatment of neurodegenerative condition of the brain or epilepsy that are characterized by neurodegeneracy. In this way, the therapeutic population of cultured cells may be administered to human patients who are either suffering from, or prone to suffer from the disease by providing compounds that inhibit neuronal degeneration. In particular, the disease is associated with neurodegenerative disorder of the brain, loss of nerve cell, particularly in the hippocampus and cerebral cortex, reduced neurotransmitters, cerebrovascular degeneration, crushed nerve in the spine, and/or loss of cognitive ability.

The formulation of therapeutic compounds is generally known in the art and reference can conveniently be made to Remington's Pharmaceutical Sciences, 17th ed., Mack Publishing Co., Easton, Pa., USA. For example, from about 0.05 µg to about 20 mg per kilogram of body weight per day may be administered. Dosage regime may be adjusted to provide the optimum therapeutic response. For example, several divided doses may be administered daily or the dose may be proportionally reduced as indicated by the exigencies of the therapeutic situation. The active compound may be administered in a convenient manner such as by the oral, intravenous (where water soluble), intramuscular, subcutaneous, intra nasal, intradermal or suppository routes or implanting (eg using slow release molecules by the intraperitoneal route or by using cells e.g. monocytes or dendrite cells sensitised *in vitro* and adoptively transferred to the recipient). Depending on the route of administration, the peptide may be required to be coated in a material to protect it from the action of enzymes, acids and other natural conditions which may inactivate said ingredients.

For example, the low lipophilicity of the peptides will allow them to be destroyed in the gastrointestinal tract by enzymes capable of cleaving peptide bonds and in the stomach by acid hydrolysis. In order to administer peptides by other than parenteral administration, they will be coated by, or administered with, a material to prevent its inactivation. For example, peptides may be administered in an adjuvant, co-administered with enzyme inhibitors or in liposomes. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEP) and trasylol. Liposomes include water-in-oil-in-water CGF emulsions as well as conventional liposomes.

The active compounds may also be administered parenterally or intraperitoneally. Dispersions can also be prepared in glycerol liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The prevention of the action of microorganisms can be brought about by various antibacterial and antifungal agents, for example, chlorobutanol, phenol, sorbic acid, theomersal and the like. In many cases, it will be preferable to include isotonic agents, for example, sugars or sodium chloride. Prolonged absorption of the injectable compositions can be brought about by the use in the composition of agents delaying absorption, for example, aluminium monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterile active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

When the peptides are suitably protected as described above, the active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compomd. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 0.1 µg and 2000 mg of active compound.

The tablets, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent" includes any and all solvents, dispersion media, coatings antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired.

The principal active ingredient is compounded for convenient and effective administration in effective amounts with a suitable pharmaceutically acceptable carrier in dosage unit form. A unit dosage form can, for example, contain the principal active compound in amounts ranging from 0.5 µg to about 2000 mg. Expressed in proportions, the active compound is generally present in from about 0.5 µg/ml of carrier. In the case of compositions containing supplementary active ingredients, the dosages are determined by reference to the usual dose and manner of administration of the said ingredients.

### Delivery Systems

Various delivery systems are known and can be used to administer a compound as described herein, e.g., encapsulation in liposomes, microparticles, microcapsules, recombinant cells capable of expressing the compound, receptor-mediated endocytosis, construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody or a peptide, care must be taken to use materials to which the protein does not absorb. In another embodiment, the compound or composition can be delivered in a vesicle, in particular a liposome. In yet another embodiment, the compound or composition can be delivered in a controlled release system. In one embodiment, a pump may be used. In another embodiment, polymeric materials can be used. In yet another embodiment, a controlled release system can be placed in proximity of the therapeutic target, i.e., the brain, thus requiring only a fraction of the systemic dose.

A composition is said to be "pharmacologically or physiologically acceptable" if its administration can be tolerated by a recipient animal and is otherwise suitable for administration to that animal. Such an agent is said to be administered in a "therapeutically effective amount" if the amount administered is physiologically significant. An agent is physiologically significant if its presence results in a detectable change in the physiology of a recipient patient.

### Gene Therapy

In a specific embodiment, nucleic acids comprising sequences encoding the TGF superfamily polypeptide are administered to treat, inhibit or prevent a disease or disorder associated with neuronal degeneration by way of gene therapy. Gene therapy refers to therapy performed by the administration to a subject of an expressed or expressible nucleic acid. In this embodiment of the invention, the nucleic acids produce their encoded protein that mediates a therapeutic effect.

For general reviews of the methods of gene therapy, see Goldspiel et al., Clinical Pharmacy 12:488-505 (1993); Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIBTECH 11(5):155-215 (1993). Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In a preferred aspect, nucleic acid sequences may encode a protein belong to TGF superfamily polypeptide, in which the nucleic acid sequences are part of expression vectors that express the polypeptides in a suitable host. In particular, such nucleic acid sequences have promoters operably linked to the polypeptide coding region, said promoter being inducible or constitutive, and, optionally, tissue-specific. In another particular embodiment, nucleic acid molecules are used in which the polypeptide coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA 86:8932-8935 (1989); Zijlstra et al., Nature 342:435-438 (1989).

Delivery of the nucleic acids into a patient may be either direct, in which case the patient is directly exposed to the nucleic acid or nucleic acid- carrying vectors, or indirect, in which case, cells are first transformed with the nucleic acids *in vitro*, then transplanted into the patient. These two approaches are known, respectively, as *in vivo* or *ex vivo* gene therapy.

In a specific embodiment, the nucleic acid sequences are directly administered *in vivo*, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retrovirals or other viral vectors, or by direct injection of naked DNA, or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem. 262:4429-4432 (1987)) (which can be used to target cell types specifically expressing the receptors) and so on. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor. Alternatively, the nucleic acid can be introduced intracellularly and incorporated within host cell DNA for expression, by homologous recombination (Koller and Smithies, Proc. Natl. Acad. Sci. USA 86:8932-8935 (1989); Zijlstra et al., Nature 342:435-438 (1989)).

In a specific embodiment, viral vectors that contain nucleic acid sequences encoding the polypeptide are used. The nucleic acid sequences encoding the polypeptide to be used in gene therapy are cloned into one or more vectors, which facilitates delivery of the gene into a patient. Retroviral vectors, adenoviral vectors and adeno-associated viruses are examples of viral vectors that may be used. Retroviral vectors contain the components necessary for the correct packaging of the viral genome and integration into the host cell DNA.

Adenoviruses are especially attractive vehicles for delivering genes to respiratory epithelia because they naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. In addition, adeno-associated virus (AAV) has also been proposed for use in gene therapy.

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion and so on. Numerous techniques are known in the art for the introduction of foreign genes into cells and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T-lymphocytes, B-lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, and so on.

In a preferred embodiment, the cell used for gene therapy is autologous to the patient. Alternatively, the patient may be autologous..

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

In particular, when NIH3T3 cells transduced with BMP-3 or BMP-4 encoding gene and was exposed to the cytotoxic agent methamphetamine, the cells died less. Further, when mice exposed to the neurotoxin kainate was treated with NIH3T3 cells transduced with BMP-4 encoding gene, neuroprotection occurred.

### EXAMPLES

### EXAMPLE I - MATERIALS AND METHODS

### Materials

Mature Sprague-Dawley strain rats (16∼18 weeks old) with weight of 400±10 g have been used for this study. The reason for using mature rats is because it is easier to observe changes due to nerve regeneration and physiological changes compared with rats in growing stage.

### Methods

It is technically difficult to inject functional protein BMP into peripheral nerves. Thus, in this study, rat fibroblasts producing BMP2, BMP4, BMP9 and Glial cell line-derived neurotrophic factor (GDNF) were directly transfected into the peripheral nerves so that BMP2, BMP9, and GDNF are secreted locally. Total of 60 rats were divided into 5 groups. Each group consisted of 12 rats with injured nerves. The first group was a control group with rats whose injured nerves were treated with non-modified fibroblast without transgene. The second group (BMP2 group) was composed of rats with injured nerves, which were treated with gene-modified fibroblast with BMP-2 transgene, and the third group (BMP4 group) was composed of rats with injured nerves, which were treated with gene-modified fibroblast with BMP-4 transgene. The fourth group (BMP9 group) was composed of rats with injured nerves, which were treated with gene-modified fibroblast with BMP-9 transgene. The fifth group (GDNF group) was composed of rats with injured nerves, which were treated with gene-modified fibroblast with GDNF transgene. Histological examination of tissues was performed 2, 4, and 8 weeks after the treatment by sacrificing 2 rats from each group and harvesting sciatic nerves from both limbs. Nerve motor conduction study was also performed with sciatic nerves from limbs by setting the original base-line value before the experiment and measuring nerve conduction every other week up to 8 weeks after the surgery.

### Nerve Injury

The white rat was anesthetized by injecting 4% chloral hydrate in a concentration of 300 mg/Kg into abdominal cavity. Hair was removed from the posterior and femoral regions of the right limb before being fixed in prone position. After sterilizing the femoral region with potadine and 70% alcohol, about 1∼1.5 cm of epidermis around the central part of the femoral region was vertically incised and femoral biceps were pulled outward to expose sciatic nerve. Nerve injuries were made according to DeKoning et al. (De Koning et al., J Neurol Sci, 1986, 74: 237-246) by incising the skin between the thigh and total knee joint to 2∼2.5 cm in length and exfoliating the posterior and total knee joint muscles to expose sciatic nerve and then injuring the nerve exposed at sciatic herniation by crushing with haemostatic forceps (Crile, 15 cm) for 30 seconds. The forceps could be set at three different levels of holding strength and to apply the same level of holding for the nerve injury at fixed areas, a black line was marked on the forceps at 5 cm from the end enabling crushing injuries at the fixed area by the strongest holding level. After removing the haemostatic forceps, the first or control group was injected with buffer with 0.05 ml non-modified fibroblast (unit: 5 x 10⁵ cell/50 µl) within 2 mm of nerve injury area using ultra-fine needle (30 gauge). The second and the third experimental groups were injected with gene-modified fibroblasts secreting BMP2,BMP4, BMP9 GDNF, respectively, by the same method before suturing and sterilizing the wounded area.

### Nerve Conduction Test

Nerve conduction test was performed after anesthetizing the rat with 4% chloral hydrate. The activity-recording electrode was placed on calf muscle to stimulate sciatic notch, the reference electrode was placed on the foot, and the ground electrode was placed between stimulating electrode and the recording electrode. Patch-like electrode was used as the recording electrode and the ground electrode was put under the skin by using a needle electrode. The nerve conduction test was done by using KeyPoint (Dantec, Denmark). Frequency, recording printing rate, and recording sensitivity in this study were set as 2∼10,000 Hz, 2 msec/division, 5 mV/division, respectively. Nerve conduction tests were performed every 2 weeks. Measurements were taken from the beginning of the experiment and followed at weeks 2, 4, 6, and 8 afterwards. Latency and amplitude during the tests were measured by the amplitude between the baseline and negative electrode point. 5 rats were selected from each group for the nerve conduction study and 10 measurements were obtained from both. Temperatures of the laboratory and the rats were maintained at 25°C and 30°C, respectively.

### Pathological/Histological Tissue Examination

Examination of the nerve tissues from rats was carried out to observe natural healing after the injury by examining normal nerves before they were injured from the beginning of the experiment. Four (4) rats (8 nerves) were used to observe regeneration of nerves with cells lacking transgene procedure. 2 rats (4 nerves) were randomly selected from each group and the tissues were examined after 2, 4, and 8 weeks. For the tissue examination, about 2 cm sciatic nerves were exfoliated from the area of crushed injury in anesthetized rats. The changes in nerve tissues were observed under optical microscopy after being fixed with buffered formaldehyde solution and then stained with hematoxylin-eosin (H & E) and modified trichrome (MT) stains.

### Data Analysis

2, 4, 6 and 8 weeks after the nerve injury, the maximum amplitude of action potential and changes during the latency of compound muscle from each group was analyzed by comparing with reference group, and statistic analysis was carried out by SPSS-PC program. Comparisons between each group were performed by ANOVA and t-test and the level of significance was set at 0.05. The degrees of injury and regeneration were determined by histological data as was interpreted by a pathologist.

### EXAMPLE II - WEIGHT CHANGES

Initially, the rats weighed in the range of 400 ± 10 g. Weight changes afterward are shown in Table 2. No difference was observed between each group at week 2, but BMP9 group showed some differences (p<0.05) compared with other groups at weeks 4 and 6. At week 8, there was no statistically significant difference between the groups (p>0.05) (Table 1).

**Table 2. Changes of Body Weight in Experimental Groups**

| | 2^{nd} week | 4^{th} week | 6^{th} week | 8^{th} week |
|---|---|---|---|---|
| Sham | 380.7 ± 29.0 | 365.0 ± 57.7 | 430.0 ± 57.7 | 460.0 ± 46.2 |
| BMP2 | 370.6 ± 24.8 | 368.3 ± 74.7 | 382.5 ± 141.5 | 470.0 ± 40.9 |
| BMP4 | 375.3 ± 23.6 | 369.3 ± 76.9 | 395.5 ± 85.5 | 490.0 ± 50.8 |
| BMP9 | 366.8 ± 34.2 | 442.0 ± 79.8 | 505.0 ± 77.6 | 508.0 ± 60.6 |
| GDNF | 378.4 ± 22.7 | 398.2 ± 64.2 | 422.3 ± 89.5 | 498.0 ± 55.9 |

### EXAMPLE III - Changes of Latency

Baseline data measured randomly from each group before the experiment showed latency of 1.44 ± 0.11 msec. Latencies after the trauma showed differences between the control group, BMP2, BMP4, BMP9 and GDNF group at weeks 2 and 4, but lacked statistical significance (p>0.05). The latencies of BMP2 and BMP9 groups were shortened significantly compared with the control group at week 6 (p<0.05). However, there was no difference in the latencies among the 5 groups at week 8 (p>0.05) (Table 3).

**Table 3. Latency Change in Groups**

| | 2^{nd} week | 4^{th} week | 6^{th} week | 8^{th} week |
|---|---|---|---|---|
| Sham | 1.30 ± 0.11 | 1.29 ± 0.04 | 1.25 ± 0.21 | 1.10 ± 0.0 |
| BMP2 | 1.24 ± 0.14 | 1.25 ± 0.07 | 1.08 ± 0.07* | 1.04 ± 0.04 |
| BMP4 | 1.25 ± 0.13 | 1.26 ± 0.08 | 1.07 ± 0.05* | 1.14 ± 0.03. |
| BMP9 | 1.19 ± 0.12 | 1.23 ± 0.10 | 1.06 ± 0.03* | 1.15 ± 0.07 |
| GDNF | 1.22 ± 0.14 | 1.24 ± 0.05 | 1.09 ± 0.06* | 1.15 ± 0.05 |

### EXAMPLE IV - Changes of Amplitude

Baseline data randomly measured from each group before the experiment showed amplitude of 23.9 ± 4.3 mV. Amplitude of BMP9 group increased significantly compared with the control group at weeks 2 and 4 after the trauma (p<0.05). BMP2 and BMP9 groups showed significant differences compared to the control group at week 6 (p<0.05) and significant differences were shown in the order of BMP9, BMP2 and the control groups at week 8 (p<0.05) (Table 4).

**Table 4. Changes of Amplitude**

| | 2^{nd} week | 4^{th} week | 6^{th} week | 8^{th} week |
|---|---|---|---|---|
| Sham | 3.98 ± 1.52 | 6.17 ± 1.27 | 6.33 ± 1.27 | 6.3 ± 1.31 |
| BMP2 | 6.14 ± 1.51 | 7.51 ± 1.29 | 9.00 ± 1.69* | 7.17 ± 0.50* |
| BMP4 | 6.54 ± 1.52 | 8.51 ± 1.31* | 8.90 ± 1.59* | 7.35 ± 0.60* |
| BMP9 | 6.79 ± 1.34* | 9.53 ± 4.47* | 9.47 ± 1.22* | 10.26 ± 2.27* |
| GDNF | 6.94 ± 1.53 | 9.51 ± 3.29 | 10.10 ± 1.69* | 11.17 ± 1.80* |

### EXAMPLE V - Histological and Pathological Observations

Histological nerve tissue examination of randomly selected rats from each group showed changes similar to the results of the nerve physiology examination. The control group showed the slowest recovery rate since axons of the nerve tissues were not regenerated and inflammatory reaction remained. The groups transfected with gene-modified fibroblasts secreting BMP2 BMP4, BMP9, or GDNF did not show any significant difference compared to the control group in the early stage, and all groups showed severe pathological indications caused by crushed damage of the nerves such as vacuolar changes and infiltration of inflammatory monocyte, bleeding of epineurium vein at week 2. For the control group, these symptoms persisted throughout 8 weeks. However, for the BMP2 group, the size of inflammation and vacuolar changes were significantly reduced and only half of them showed these symptoms at weeks 4 and 8. For the BMP4, BMP9 or GDNF group, these effects were more prominent at weeks 4 and 8, and only one third of them showed loss of axon, vacuolar changes. Inflammation was very mild.

### EXAMPLE VI

### Materials

Mature Sprague-Dawley strain rats (16∼18 weeks old) with weight of 400±10 g have been used for this study. The reason for using mature rats is because it is easier to observe changes due to nerve regeneration and physiological changes compared with rats in growing stage.

### Methods

To evade the possibility of cancer formation, irradiated schwann cells were injected to get maximum as well as safer results. Thus, in this study, rat schwann cells producing BMP2, BMP4, and GDNF were irradiated with 15Gy strength and were directly transfected into the peripheral nerves so that BMP2 BMP4, and GDNF are secreted locally. Total of 60 rats were divided into 5 groups. Each group consisted of 12 rats with injured nerves. The first group was a control group with rats whose injured nerves were treated with non-modified fibroblast without transgene. The second group (BMP2 group) was composed of rats with injured nerves, which were treated with gene-modified fibroblast with BMP-2 transgene, and the third group (BMP4 group) was composed of rats with injured nerves, which were treated with gene-modified fibroblast with BMP-4 transgene. The forth group (GDNF group) was composed of rats with injured nerves, which were treated with gene-modified fibroblast with GDNF transgene Histological examination of tissues was performed 2, 4, and 8 weeks after the treatment by sacrificing 2 rats from each group and harvesting sciatic nerves from both limbs.

### Nerve Injury

The white rat was anesthetized by injecting 4% chloral hydrate in a concentration of 300 mg/Kg into abdominal cavity. Hair was removed from the posterior and femoral regions of the right limb before being fixed in prone position. After sterilizing the femoral region with potadine and 70% alcohol, about 1∼1.5 cm of epidermis around the central part of the femoral region was vertically incised and femoral biceps were pulled outward to expose sciatic nerve. Nerve injuries were made according to DeKoning et al. (De Koning et al., J Neurol Sci, 1986, 74: 237-246) by incising the skin between the thigh and total knee joint to 2∼2.5 cm in length and exfoliating the posterior and total knee joint muscles to expose sciatic nerve and then injuring the nerve exposed at sciatic herniation by crushing with haemostatic forceps (Crile, 15 cm) for 30 seconds. The forceps could be set at three different levels of holding strength and to apply the same level of holding for the nerve injury at fixed areas, a black line was marked on the forceps at 5 cm from the end enabling crushing injuries at the fixed area by the strongest holding level. After removing the haemostatic forceps, the first or control group was injected with buffer with 0.05 ml non-modified fibroblast (unit: 5 x 10⁵ cell/50 µl) within 2 mm of nerve injury area using ultra-fine needle (30 gauge). The second and the third experimental groups were injected with gene-modified fibroblasts secreting BMP2, BMP4, GDNF, respectively, by the same method before suturing and sterilizing the wounded area.

### Peripheral Nerve Test

Measurements were taken from the beginning of the experiment and followed at weeks 2, 4, 6, and 8 afterwards. Latency and threshold during the tests were measured by the Randall Selitto for mechanical threshold, or hot water bath (49°C) for thermal latency. Temperatures of the laboratory and the rats were maintained at 25°C and 30°C, respectively.

### Data Analysis

2, 4, 6 and 8 weeks after the nerve injury, the maximum latency and threshold from each group was analyzed by comparing with reference group, and statistic analysis was carried out by SPSS-PC program. Comparisons between each group were performed by ANOVA and t-test and the level of significance was set at 0.05.

### EXAMPLE VII - Changes of Thermal Latency

Baseline data measured randomly from each group before the experiment showed latency of 12.44 ± 3.13 sec. Latencies after the trauma showed differences between the control group, BMP2, BMP4, and GDNF group. The latencies of BMP2, BMP4, and GDNF groups were shortened significantly compared with the control group at week 6 (p<0.05) (Table 5).

**Table 5. Latency Change in Groups**

| | 3^{rd} Day | 6^{th} Day | 9^{th} Day | 12^{th} Day |
|---|---|---|---|---|
| Sham | 11.25 ± 1.17 | 12.29 ± 1.04 | 12.85 ± 1.21 | 11.10 ± 2.09 |
| BMP2 | 10.28 ± 2.15 | 8.27 ± 3.05 | 7.08 ± 1.07* | 8.04 ± 3.34 |
| BMP4 | 11.17 ± 1.16 | 10.28 ± 2.56 | 9.07 ± 2.95 | 7.14 ± 2.23* |
| GDNF | 7.25 ± 1.15* | 6.52 ± 2.18* | 7.09 ± 2.06* | 6.15 ± 2.05* |

### EXAMPLE VIII - Changes of Mechanical threshold

Baseline data randomly measured from each group before the experiment showed threshold of 12.1 ± 1.0 g. Threshold of GDNF group decreased significantly compared with the control group at Day 6 and 9 after the trauma (p<0.05). BMP2 and BMP4 groups showed significant differences compared to the control group at Day 9 (p<0.05) (Table 6).

**Table 6. Changes of Amplitude**

| | 3^{rd} Day | 6^{th} Day | 9^{th} Day | 12^{th} Day |
|---|---|---|---|---|
| Sham | 10.59 ± 1.52 | 9.37 ± 1.57 | 10.31 ± 1.20 | 11.3 ± 1.01 |
| BMP2 | 9.14 ± 1.51 | 7.41 ± 1.19 | 7.00 ± 1.39* | 10.17 ± 0.50 |
| BMP4 | 8.54 ± 1.52 | 8.61 ± 1.01 | 6.90 ± 1.54* | 9.35 ± 0.40 |
| GDNF | 6.84 ± 1.01* | 6.31 ± 1.19* | 5.10 ± 1.09* | 8.17 ± 1.80 |

### EXAMPLE IX - Histological and Pathological Observations

Histological nerve tissue examination of randomly selected rats from each group showed changes similar to the results of the nerve physiology examination. The control group showed the slowest recovery rate since axons of the nerve tissues were not regenerated and inflammatory reaction remained. The groups transfected with gene-modified fibroblasts secreting BMP2 BMP4, or GDNF did not show any significant difference compared to the control group in the early stage, and all groups showed severe pathological indications caused by crushed damage of the nerves such as vacuolar changes and infiltration of inflammatory monocyte, bleeding of epineurium vein at week 2. For the control group, these symptoms persisted throughout 8 weeks. However, for the BMP2 group, the size of inflammation and vacuolar changes were significantly reduced and only half of them showed these symptoms at weeks 4 and 8. For the BMP4, BMP9 or GDNF group, these effects were more prominent at weeks 4 and 8, and only one third of them showed loss of axon, vacuolar changes. Inflammation was very mild.

### EXAMPLE X - Neuroprotective effects of BMP-3 in response to methamphetamine (MAP)-induced cytotoxicity

DMEM was used as a culture medium for NIH 3T3 cell (control), BMP3 or BMP4. Exposure to a high dose (1mM) of methamphetamine (MAP) for 24 hrs induced significant cytotoxicity in 3T3 cell (See Fig. 1 hatched bar over "3T3" Group; and representative photograph D. Cell viability was approximately 40 % as compared with 3T3 cell alone (Fig. 1, closed bar over "3T3"; and representative photograph A. Cell viability was approximately 100 %. 3T3 + MAP vs. 3T3 alone, P < 0.01 (Students' t-test).

MAP-induced cytotoxicity was not significantly observed in the treatment of BMP-3 plus MAP (See Fig. 1, closed and hatched bars over "3T3-PMT-BMP3" Group; representative photograph B shows 3T3/BMP-3 alone, and representative photograph E shows 3T3/BMP-3 exposed to MAP. 3T3 + MAP vs. 3T3/BMP3 + MAP, P < 0.01 (Students' t-test).

However, BMP4 did not prevent MAP-induced cytotoxicity (See Fig. 1, closed and hatched bars over the "3T3-hBMP4" Group. The closed bar indicates 3T3/BMP-4 alone; representative photograph C. The hatched bar indicates 3T3/BMP-4 cell treated with MAP; representative photograph F. 3T3/BMP4 alone vs. 3T3/BMP4 + MAP, P < 0.01 (Students' t-test). The cell population / each well was about 300,000.

### EXAMPLE XI - BMP4 treatment significantly prevented kainate-induced neuronal degeneration in mice

Kainate (KA) is an excitoneurotoxin, which is well-recognized as a model of human temperal lobe epilepsy. In addition, it is a useful tool *in vivo* for measurement on the neurodegenerative change. KA-induced neuronal loss is via KA receptor activation. KA receptor located mainly in the CA3 region of the hippocampus. Animals receiving saline solution showed an intact pyramidal cell layer (cellular architecture) in the hippocampus. Animals receiving saline solution plus KA exhibited a significant neuronal loss (0.001 vs. Control/Saline) in the CA3 area (cell population of left-sided C-like area was significantly reduced). Intracerebroventricular injection (I.C.V.) with 3T3 did not affect KA (I.C.V)-induced neuronal loss of the CA3 area. However, I.C.V. with 3T3 cells recombinantly expressing BMP4 about 6 hrs before KA (I.C.V.) significantly attenuated (P < 0.01 vs. Sal + KA) neuronal loss within the CA 3 area. Therefore BMP 4 is a protective factor in response to KA-induced hippocampal degeneration.

## Claims

1. Population of cultured cells transfected *in vitro* with a generated recombinant viral or plasmid vector comprising a DNA sequence encoding a bone morphogenetic protein BMP operatively linked to a promoter, for use in treating a neurodegenerative condition of the brain, or epilepsy, by protecting degeneration of nerve, wherein treatment comprises injecting the transfected cells to an area near an injured nerve, such that expression of the DNA sequence within the area near the injured nerve causes attenuation of degeneration of the nerve.

2. Population of cultured cells for use according to claim 1, wherein the BMP is BMP-2, BMP-3, BMP-4 or BMP-9.

3. Population of cultured cells for use according to claim 1, wherein the cell is a connective tissue cell.

4. Population of cultured cells for use according to claim 3, wherein the cell is a fibroblast cell.

5. Population of cultured cells for use according to claim 1, wherein the cell is a nerve cell.

6. Population of cultured cells for use according to claim 5, wherein the cell is a glial cell.

7. Population of cultured cells for use according to claim 1, wherein the cell is irradiated.

8. Population of cultured cells for use according to claim 1, wherein the vector is a viral vector,

9. Population of cultured cells for use according to claim 6, wherein the vector is retroviral vector, adeno-associated viral vector, adenoviral vector, or herpes viral vector.

## Patentansprüche

1. Population von kultivierten Zellen, die *in vitro* mit einem erzeugten rekombinanten viralen oder Plasmid-Vektor transfiziert sind, umfassend eine DNA-Sequenz, die ein Knochen morphogenetisches Protein BMP codiert, das operativ mit einem Promotor verbunden ist, zur Verwendung bei der Behandlung eines neurogenerativen Zustands des Gehirns oder von Epilepsie durch Schutz vor Nervendegeneration, wobei die Behandlung das Injizieren der transfizierten Zellen in einen Bereich nahe eines verletzten Nervs umfasst, so dass die Expression der DNA-Sequenz in dem Bereich nahe des verletzten Nervs eine Verminderung der Nervendegeneration verursacht.

2. Population von kultivierten Zellen zur Verwendung gemäß Anspruch 1, wobei das BMP BMP-2, BMP-3, BMP-4 oder BMP-9 ist.

3. Population von kultivierten Zellen zur Verwendung gemäß Anspruch 1, wobei die Zelle eine Bindegewebszelle ist.

4. Population von kultivierten Zellen zur Verwendung gemäß Anspruch 3, wobei die Zelle eine Fibroblastenzelle ist.

5. Population von kultivierten Zellen zur Verwendung gemäß Anspruch 1, wobei die Zelle eine Nervenzelle ist.

6. Population von kultivierten Zellen zur Verwendung gemäß Anspruch 5, wobei die Zelle eine Gliazelle ist.

7. Population von kultivierten Zellen zur Verwendung gemäß Anspruch 1, wobei die Zelle bestrahlt ist.

8. Population von kultivierten Zellen zur Verwendung gemäß Anspruch 1, wobei der Vektor ein viraler Vektor ist.

9. Population von kultivierten Zellen zur Verwendung gemäß Anspruch 6, wobei der Vektor ein retroviraler Vektor, adeno-assoziierter viraler Vektor, adenoviraler Vektor oder Herpes viraler Vektor ist.

## Revendications

1. Population de cellules en culture transfectées *in vitro* avec un vecteur viral ou plasmidique recombinant généré comprenant une séquence d'ADN codant pour une protéine morphogénétique osseuse BMP en liaison fonctionnelle avec un promoteur, destinée à être utilisée dans le traitement d'une maladie neurodégénérative du cerveau, ou de l'épilepsie, en protégeant la dégénérescence d'un nerf, où le traitement comprend l'injection des cellules transfectées dans une région à proximité d'un nerf lésé, de sorte que l'expression de la séquence d'ADN dans la région à proximité du nerf lésé entraîne une atténuation de la dégénérescence du nerf.

2. Population de cellules en culture destinée à être utilisée selon la revendication 1, dans laquelle la BMP est la BMP-2, BMP-3, BMP-4 ou BMP-9.

3. Population de cellules en culture destinée à être utilisée selon la revendication 1, dans laquelle la cellule est une cellule du tissu conjonctif.

4. Population de cellules en culture destinée à être utilisée selon la revendication 3, dans laquelle la cellule est une cellule de fibroblaste.

5. Population de cellules en culture destinée à être utilisée selon la revendication 1, dans laquelle la cellule est une cellule nerveuse.

6. Population de cellules en culture destinée à être utilisée selon la revendication 5, dans laquelle la cellule est une cellule gliale.

7. Population de cellules en culture destinée à être utilisée selon la revendication 1, dans laquelle la cellule est irradiée.

8. Population de cellules en culture destinée à être utilisée selon la revendication 1, dans laquelle le vecteur est un vecteur viral.

9. Population de cellules en culture destinée à être utilisée selon la revendication 6, dans laquelle le vecteur est un vecteur rétroviral, un vecteur viral adéno-associé, un vecteur adénoviral, ou un vecteur herpèsvirus.
